# EUROPÄISCHE PATENTSCHRIFT

(11) **EP 2 854 950 B1**
(45) Veröffentlichungstag und Bekanntmachung des Hinweises auf die Patenterteilung: **14.12.2016**
(21) Anmeldenummer: 13725683.0
(22) Anmeldetag: 29.05.2013
(51) Int. Cl.: A61Q 5/02, A61Q 19/10, A61K 8/46, A61K 8/44, A61K 8/42, C11D 1/52

(54) **VERWENDUNG VON N-METHYL-N-ACYLGLUCAMINEN ALS KÄLTESTABILISATOREN IN TENSIDLÖSUNGEN**
USE OF N-METHYL-N-ACYLGLUCAMINES AS COLD STABILIZERS IN SURFACTANT SOLUTIONS
UTILISATION DE N-MÉTHYL-N-ACYLGLUCAMINES COMME STABILISATEURS AU FROID DANS DES SOLUTIONS TENSIOACTIVES

(30) Priorität: 30.05.2012 DE 102012010700
(43) Veröffentlichungstag der Anmeldung: 08.04.2015
(73) Patentinhaber: Clariant International Ltd, 4132 Muttenz (CH)
(72) Erfinder: KLUG, Peter, 63762 Grossostheim (DE); MILDNER, Carina, 65931 Frankfurt am Main (DE)
(74) Vertreter: Holmes, Rosalind
(86) Internationale Anmeldenummer: PCT/EP2013/061046
(87) Internationale Veröffentlichungsnummer: WO 2013/178670

(56) Entgegenhaltungen:
- WO-A1-98/00496

## Beschreibung

Die Erfindung betrifft die Verwendung von N-Methyl-N-acylglucaminen als Kältestabilisatoren in wässrigen Tensidlösungen.

Es ist bekannt, kurzkettige Zuckertenside als Solubilisatoren in oberflächenaktiven Mitteln einzusetzen.

Bei der Herstellung von flüssigen oberflächenaktiven Mitteln wie Haarshampoos, Geschirrspül- oder Flüssigwaschmitteln tritt häufig das Problem auf, dass die tensidischen Inhaltsstoffe keine ausreichende Wasserlöslichkeit aufweisen und insbesondere in Gegenwart von Salzen austrüben und mehrere Phasen bilden. Zur Solubilisierung der tensidischen Inhaltsstoffe in oberflächenaktiven Mitteln wie Wasch-, Spül- und Reinigungsmitteln sowie in kosmetischen oder pharmazeutischen Zubereitungen offenbart WO 96/14374 Carbonsäure-N-alkyl-N-polyhydroxyalkylamide der Formel

R²CO-NR³-[Z],

in der R²CO für einen aliphatischen Acylrest mit 1 bis 8 C-Atomen, R³ für Wasserstoff oder einen Alkyl- oder Hydroxyalkylrest mit 1 bis 8 C-Atomen und [Z] für einen Polyhydroxyalkylrest mit 3 bis 12 C-Atomen und 3 bis 10 OH-Gruppen steht. Als bevorzugt werden Carbonsäure-N-alkylglucamine genannt, in denen R²CO für den Acylrest der Ameisensäure, Essigsäure, Propionsäure, Buttersäure oder Capronsäure steht und der Alkylrest R³ für Methyl oder Octyl steht. In den Beispielen werden Essigsäure-N-octylglucamin, Buttersäure-N-octylglucamin und Capronsäure-N-methylglucamin als Solubilisatoren für ein Gemisch enthaltend Ölsäuresulfonat-Natriumsalz, Kokosfettalkoholethersulfat-Natriumsalz und Kokosfettsäure-Triethanolammoniumsalz verwendet.

WO 95/17880 offenbart eine Haarshampoo-Zusammensetzung enthaltend Alkylglykolethersulfate und Alkylsulfate sowie Polyhydroxyalkylfettsäureamide. Als Alkylglykolethersulfat wird u.a. Lauryltriethylenglykolethersulfat, als Alkylsulfat wird u.a. Laurylsulfat genannt. Als Polyhydroxyalkylfettsäureamide werden Verbindungen der allgemeinen Formel

R²-CO-NR¹-Z

aufgeführt, wobei R¹ vorzugsweise C₁-C₄-Alkyl, insbesondere Methyl, R² vorzugsweise geradkettiges C₇-C₁₉-Alkyl oder -Alkenyl, insbesondere geradkettiges C₁₁-C₁₆-Alkyl oder -Alkenyl, und Z insbesondere 1-Deoxyglucityl, 2-Deoxyfructityl, 1-Deoxymaltityl, 1-Deoxylactityl, 1-Deoxygalactityl, 1-Deoxymannityl oder 1-Deoxymaltotriothityl ist. Die Beispiele offenbaren Haarshampoo-Zusammensetzungen enthaltend Ammoniumlaurylsulfat, Ammoniumlauryltriethylenglykolsulfat und Lauryl-N-methylglucamin.

Bei wässrigen Tensidlösungen enthaltend lineare Alkylsulfate und/oder Alkylethersulfate einerseits sowie Betain-Tenside andererseits verringert sich bei sinkender Temperatur die Viskosität. Dies kann auf ein Ausfallen der Tenside aus der Lösung bei niedrigen Temperaturen zurückgeführt werden. Dieser Effekt ist in der Praxis als Sporttaschen-Effekt bekannt und unerwünscht, da Körper- und Haarreinigungsmittel bei Lagerung in der Kälte wasserdünn werden und nicht mehr das erwünschte Handhabungsprofil haben.

Aufgabe der Erfindung ist es, einen Stabilisator zur Verbesserung der Kältestabilität von wässrigen Tensidlösungen bereitzustellen.

Gelöst wird die Aufgabe durch die Verwendung von N-Methyl-N-acylglucaminen, wobei mindestens 90 Gew.-% der N-Methyl-N-acylglucamine eine C₈-Acyl- oder C₁₀-Acylgruppe aufweisen, als Kältestabilisatoren in wässrigen Tensidlösungen, die ein oder mehrere anionische Tenside aus der Gruppe bestehend aus Alkylsulfaten und Alkylethersulfaten enthalten, sowie Betain-Tenside enthalten.

N-Methyl-N-acylglucamine weisen die allgemeine Formel (I) auf, 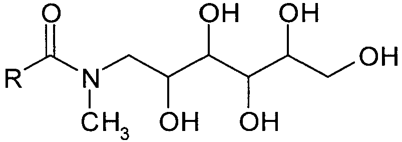 worin R einen entsprechenden Alkyl- oder ein- oder mehrfach ungesättigten Alkenylrest bedeutet, im Falle der C₈- oder C₁₀- Acylglucamine also einen C₇- bzw. C₉-Alkyl- oder ein oder mehrfach ungesättigten Alkenylrest.

Es wurde gefunden, dass in Gegenwart der erfindungsgemäß verwendeten N-Methyl-N-C₈-C₁₀-acylglucamine die Viskosität der wässrigen Tensidlösungen auch bei niedrigen Temperaturen noch überraschend hoch ist.

Die erfindungsgemäß verwendeten N-Methyl-N-acylglucaminen bestehen zu mindestens 90 Gew.-% aus N-Methyl-N-acylglucaminen, die eine C₈- oder C₁₀-Acylgruppe enthalten. Besonders bevorzugt liegt der Anteil an N-Methyl-N-acylglucaminen, die eine C₈ oder C₁₀-Acylgruppe enthalten, bei mindestens 95 Gew.-%. Daneben enthalten die erfindungsgemäß als Solubilisatoren verwendeten N-Methyl-N-acylglucamine geringe Anteile an von kurzkettigen und/oder langkettigen Fettsäuren abgeleiteten N-Methyl-N-acylglucaminen, insbesondere solche, welche C₁-C₄-Acyl, C₆-, C₁₂-, C₁₄-, C₁₆-, C₁₈- und/oder C₂₀-Acyl enthalten.

Die N-Methyl-N-acylglucamine können, wie in EP 0 550 637 B1 beschrieben, durch Umsetzung der entsprechenden Fettsäureester bzw. Fettsäureestergemische mit N-Methylglucamin in Gegenwart eines Hydroxylgruppen oder Alkoxylgruppen aufweisenden Lösungsmittels hergestellt werden. Geeignete Lösungsmittel sind beispielsweise C₁-C₄-Monoalkohole, Ethylenglykol, Propylenglykol, Glycerin sowie alkoxylierte Alkohole. Bevorzugt ist 1,2-Propylenglykol. N-Methylglucamin kann, wie ebenfalls in EP 0 550 637 A1 beschrieben, durch reduktive Aminierung von Glukose mit Methylamin erhalten werden.

Geeignete Fettsäureester, die mit den N-Methylglucaminen zu N-Methyl-N-acylglucaminen umgesetzt werden, sind im Allgemeinen die Methylester, die durch Umesterung aus natürlichen Fetten und Ölen, beispielsweise den Triglyceride, gewonnen werden.

Geeignete Rohstoffe für die Herstellung der Fettsäuremethylester sind beispielsweise Kokosöl oder Palmöl.

Im Allgemeinen enthalten die wässrigen Tensidlösungen sowohl anionische Tenside als auch Betaintenside.
Die wässrigen Tensidlösungen enthalten ein oder mehrere anionische Tenside aus der Gruppe der Alkylsulfate und Alkylethersulfate.

Bevorzugte Alkylsulfate sind die C₈-C₂₀-Alkylsulfate, insbesondere die linearen C₈-C₂₀-Alkylsulfate in Form ihrer Natrium-, Kalium- oder Ammoniumsalze. Beispiele für Alkylsulfate sind Laurylsulfat, Cocosalkylsulfat und Talgalkylsulfat. Besonders bevorzugt ist Laurylsulfat.

Bevorzugte Alkylethersulfate sind die C₈-C₂₀-Alkylethersulfate, besonders bevorzugt sind die linearen C₈-C₂₀-Alkylethersulfate, insbesondere die von den ethoxylierten Fettalkoholen abgeleiteten Alkylglykolethersulfate, in Form ihrer Natrium-, Kalium- oder Ammoniumsalze. Beispiele für Alkylethersulfate sind Laurylethersulfat, Cocosalkylethersulfat und Talgalkylethersulfat. Beispiele für Glykolethersulfate sind Lauryltriethylenglykolethersulfat, Cocosalkyltriethylenglykolethersulfat und Talgalkylhexaethylenglykolethersulfat. Insbesondere bevorzugt ist Laurylglykolethersulfat, beispielsweise Lauryldiethylenglykolethersulfat und Lauryltriethylenglykolethersulfat, insbesondere in Form der Natriumsalze.

Die wässrigen Tensidlösungen enthalten neben dem anionischen Tensid ein Betain-Tensid.

Betain-Tenside enthalten im selben Molekül eine kationische Gruppe, insbesondere eine Ammonium-Gruppe, und eine anionische Gruppe, die eine Carboxylat-Gruppe, SulfatGruppe oder Sulfonat-Gruppe sein kann. Geeignete Betaine sind Alkylbetaine wie Cocobetain oder Fettsäurealkylamidopropylbetaine, beispielsweise Cocosacylamidopropyldimethylbetain, C₁₂-C₁₈-Dimethylaminohexanoate oder C₁₀-C₁₈-Acylamidopropandimethylbetaine.

In einer bevorzugten Ausführungsform der Erfindung enthalten die wässrigen Tensidlösungen ein oder mehrere Amidopropylbetaine der allgemeinen Formel (I) 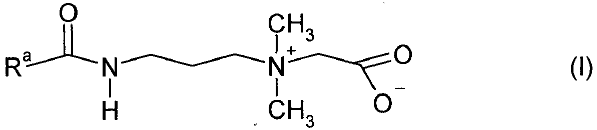 worin R^{a} eine lineare oder verzweigte gesättigte C₇-C₂₁-Alkylgruppe oder eine lineare oder verzweigte ein- oder mehrfach ungesättigte C₁-C₂₁-Alkenylgruppe ist.

In einer weiteren bevorzugten Ausführungsform der Erfindung enthalten die Tensidlösungen ein oder mehrere Betaine der Formel (II) 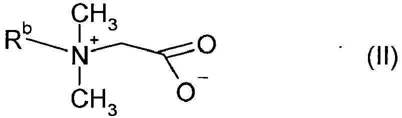 worin R^{b} eine lineare oder verzweigte gesättigte C₈-C₂₂-Alkylgruppe oder eine lineare oder verzweigte ein- oder mehrfach ungesättigte C₈-C₂₂-Alkenylgruppe ist.

In einer weiteren bevorzugten Ausführungsform der Erfindung enthalten die Tensidlösungen ein oder mehrere Sulfobetaine der Formel (III) 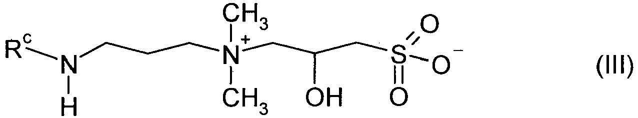 worin R^{c} eine lineare oder verzweigte gesättigte C₈-C₂₂-Alkylgruppe oder eine lineare oder verzweigte ein- oder mehrfach ungesättigte C₈-C₂₂-Alkenylgruppe ist.

Besonders bevorzugt enthalten die Tensidlösungen ein oder mehreren Betaintenside ausgewählt aus der Gruppe der Verbindungen bestehend aus den Amidopropylbetainen der Formel (I), den Betainen der Formel (II) und den Sulfobetainen der Formel (III).

In einer insbesondere bevorzugten Ausführungsform der Erfindung enthalten die Tensidlösungen ein oder mehrere Betaintenside ausgewählt aus den Amidopropylbetainen der Formel (I).

In einer weiteren insbesondere bevorzugten Ausführungsform der Erfindung enthalten die Tensidlösungen ein oder mehrere Betaintenside ausgewählt aus den Betainen der Formel (II).

In einer weiteren insbesondere bevorzugten Ausführungsform der Erfindung enthalten die Tensidlösungen ein oder mehrere Betaintenside ausgewählt aus den Sulfobetainen der Formel (III).

Vorzugsweise ist der Rest R^{a} in dem einen oder den mehreren Amidopropylbetainen der Formel (I) eine lineare oder verzweigte gesättigte C₇-C₁₇-Alkylgruppe. Unter den linearen und verzweigten gesättigten Alkylgruppen R^{a} sind die linearen gesättigten Alkylgruppen bevorzugt.

Besonders bevorzugt handelt es sich bei den Amidopropylbetainen der Formel (I) um Cocamidopropylbetaine.

Vorzugsweise ist der Rest R^{b} in dem einen oder den mehreren Betainen der Formel (II) eine lineare oder verzweigte gesättigte C₈-C₁₈-Alkylgruppe und besonders bevorzugt eine lineare oder verzweigte gesättigte C₁₂-C₁₈-Alkylgruppe. Unter den linearen und verzweigten gesättigten Alkylgruppen R^{b} sind die linearen gesättigten Alkylgruppen bevorzugt.

Vorzugsweise ist der Rest R^{c} in der einen oder den mehreren Sulfobetainen der Formel (III) eine lineare oder verzweigte gesättigte C₈-C₁₈-Alkylgruppe und besonders bevorzugt eine lineare oder verzweigte gesättigte C₁₂-C₁₈-Alkylgruppe. Unter den linearen und verzweigten gesättigten Alkylgruppen R^{c} sind die linearen gesättigten Alkylgruppen bevorzugt.

Besonders bevorzugt enthalten die wässrigen Tensidlösungen Amidopropylbetaine der Formel (I) und/oder Alkylbetaine der Formel (II).

Gegenstand der Erfindung sind auch Zusammensetzungen enthaltend
(a) N-Methyl-N-acylglucamine, wobei mindestens 90 Gew.-% der N-Methyl-N-acylglucamine eine C₈-Acyl- oder C₁₀-Acylgruppe aufweisen, als Komponente (A),
(b) anionische Tenside aus der Gruppe bestehend aus Alkylsulfaten und Alkylethersulfaten als Komponente (B),
(c) Betain-Tenside als Komponente (C),
(d) gegebenenfalls weitere Tenside als Komponente (D),
(e) Wasser als Komponente (E), sowie
(f) gegebenenfalls weitere Additive wie Konservierungsmittel, Duftstoffe, Farbstoffe, und Überfettungsmittel als Komponente (F).

Im Allgemeinen enthalten die Zusammensetzungen
(a) 0,01 bis 5,0 Gew.-%, bevorzugt 0,1 bis 3,0 Gew.-% der Komponente (A),
(b) 1,0 bis 20,0 Gew.-%, bevorzugt 5,0 bis 15 Gew.-% der Komponente (B),
(c) 0,1 bis 10 Gew.-%, bevorzugt 1,0 bis 10,0 Gew.-% der Komponente (C),
(d) 0 bis 5,0 Gew.-%, bevorzugt 0 bis 3,0 Gew.-% der Komponente (D),
(e) 55,0 bis 98,89 Gew.-%, bevorzugt 75 bis 95 Gew.-% der Komponente (E),
(f) 0 bis 5,0 Gew.-%, bevorzugt 0 bis 2,0 Gew.-% der Komponente (F).

Vorzugsweise enthalten die erfindungsgemäßen kosmetischen Zusammensetzungen die oben beschriebenen Alkylsulfate und Alkylethersulfate.

Optionale weitere Tenside (D) können kationische, nichtionische oder amphotere Tenside sein.

Geeignete kationische Tenside sind substituierte oder unsubstituierte geradkettige oder verzweigte quartäre Ammoniumsalze vom Typ R¹N(CH₃)₃X, R¹R²N(CH₃)₂X, R¹R²R³N(CH₃)X oder R¹R²R³R⁴NX. Die Reste R¹, R², R³ und R⁴ können vorzugsweise unabhängig voneinander unsubstituiertes Alkyl mit einer Kettenlänge zwischen 8 und 24 C-Atomen, insbesondere zwischen 10 und 18 C-Atomen, Hydroxyalkyl mit 1 bis 4 C-Atomen, Phenyl, C₂- bis C₁₈-Alkenyl, C₇- bis C₂₄-Aralkyl, (C₂H₄O)ₓH, wobei x von 1 bis 3 bedeutet, ein oder mehrere Estergruppen enthaltende Alkylreste oder cyclische quartäre Ammoniumsalze sein. X ist ein geeignetes Anion. Bevorzugt sind (C₈-C₂₂)-Alkyltrimethylammoniumchlorid oder -bromid, besonders bevorzugt Cetyltrimethylammoniumchlorid oder -bromid, Di-(C₈-C₂₂)-Alkyldimethylammoniumchlorid oder -bromid, (C₈-C₂₂)-Alkyldimethylbenzylammoniumchlorid oder -bromid, (C₈-C₂₂)-Alkyldimethylhydroxyethylammoniumchlorid, -phosphat, -sulfat, -lactat, besonders bevorzugt Distearyldimethylammoniumchlorid, Di(C₈-C₂₂)-Alkylamidopropyltrimethylammoniumchlorid und -methosulfat.

Als nichtionische Tenside kommen beispielsweise folgende Verbindungen in Frage:
- Polyethylen-, Polypropylen- und Polybutylenoxidkondensate von Alkylphenolen. Diese Verbindungen umfassen die Kondensationsprodukte von Alkylphenolen mit einer C₆- bis C₂₀-Alkylgruppe, die entweder linear oder verzweigt sein kann, mit Alkenoxiden. Diese Tenside werden als Alkylphenolalkoxylate, z. B. Alkylphenolethoxylate, bezeichnet.
- Kondensationsprodukte von aliphatischen Alkoholen mit 1 bis 25 mol Ethylenoxid. Die Alkyl- oder Alkenylkette der aliphatischen Alkohole kann linear oder verzweigt, primär oder sekundär sein, und enthält im Allgemeinen 8 bis 22 Kohlenstoffatome. Besonders bevorzugt sind die Kondensationsprodukte von C₁₀- bis C₂₀-Alkoholen mit 2 bis 18 mol Ethylenoxid pro mol Alkohol. Die Alkoholethoxylate können eine enge ("Narrow Range Ethoxylates") oder eine breite Homologenverteilung des Ethylenoxides ("Broad Range Ethoxylates") aufweisen. Beispiele von kommerziell erhältlichen nichtionischen Tensiden dieses Typs sind Tergitol^{®} 15-S-9 (Kondensationsprodukt eines linearen sekundären C₁₁-C₁₅-Alkohols mit 9 mol Ethylenoxid), Tergitol^{®} 24-L-NMW (Kondensationsprodukt eines linearen primären C₁₂-C₁₄-Alkohols mit 6 mol Ethylenoxid bei enger Molgewichtsverteilung). Ebenfalls unter diese Produktklasse fallen die Genapol^{®}-Marken der Clariant.
- Kondensationsprodukte von Ethylenoxid mit einer hydrophoben Basis, gebildet durch Kondensation von Propylenoxid mit Propylenglykol. Der hydrophobe Teil dieser Verbindungen weist bevorzugt ein Molekulargewicht zwischen 1500 und 1800 auf. Die Anlagerung von Ethylenoxid an diesen hydrophoben Teil führt zu einer Verbesserung der Wasserlöslichkeit. Das Produkt ist flüssig bis zu einem Polyoxyethylengehalt von ca. 50 % des Gesamtgewichtes des Kondensationsproduktes, was einer Kondensation mit bis zu ca. 40 mol Ethylenoxid entspricht. Kommerziell erhältliche Beispiele dieser Produktklasse sind die Pluronic^{®}-Marken der BASF und die Genapol^{®} PF-Marken der Clariant.
- Kondensationsprodukte von Ethylenoxid mit einem Reaktionsprodukt von Propylenoxid und Ethylendiamin. Die hydrophobe Einheit dieser Verbindungen besteht aus dem Reaktionsprodukt von Ethylendiamin mit überschüssigem Propylenoxid und weist im Allgemeinen ein Molekulargewicht von 2500 bis 3000 auf. An diese hydrophobe Einheit wird Ethylenoxid bis zu einem Gehalt von 40 bis 80 Gew.-% Polyoxyethylen und einem Molekulargewicht von 5000 bis 11000 addiert. Kommerziell erhältliche Beispiele dieser Verbindungsklasse sind die Tetronic^{®}-Marken der BASF und die Genapol^{®} PN-Marken der Clariant.

Weitere geeignete nichtionische Tenside sind Alkyl- und Alkenyloligoglycoside sowie Fettsäurepolyglykolester oder Fettaminpolyglykolester mit jeweils 8 bis 20, vorzugsweise 12 bis 18 C-Atomen im Fettalkylrest, Alkyloligoglycoside, Alkenyloligoglycoside und Fettsäure-N-alkylglucamide.

Weiterhin können die erfindungsgemäßen Zusammensetzungen amphotere Tenside enthalten. Diese können beschrieben werden als Derivate langkettiger sekundärer oder tertiärer Amine, die über eine Alkylgruppe mit 8 bis 18 C-Atomen verfügen und bei denen eine weitere Gruppe substituiert ist mit einer anionischen Gruppe, die die Wasserlöslichkeit vermittelt, so z. B. mit einer Carboxyl-, Sulfat- oder Sulfonat-Gruppe. Bevorzugte Amphotenside sind N-(C₁₂-C,₈)-Alkyl-β-aminopropionate und N-(C₁₂-C₁₈)-Alkyl-β-iminodipropionate als Alkali- und Mono-, Di- und Trialkylammonium-Salze. Geeignete weitere Tenside sind auch Aminoxide. Es sind dies Oxide tertiärer Amine mit einer langkettigen Gruppe von 8 bis 18 C-Atomen und zwei meist kurzkettigen Alkylgruppen mit 1 bis 4 C-Atomen. Bevorzugt sind hier beispielsweise die C₁₀- bis C₁₈-Alkyldimethylaminoxide, Fettsäureamidoalkyldimethylaminoxid.

Weitere Additive (F) sind beispielsweise Konservierungsmittel, Duftstoffe, Farbstoffe und Rückfettungsmittel.

Als Konservierungsmittel eignen sich die in dem betreffenden Anhang der europäischen Kosmetikgesetzgebung gelisteten Konservierungsmittel, beispielsweise Phenoxyethanol, Benzylalkohol, Parabene, Benzoesäure und Sorbinsäure, besonders gut geeignet ist beispielsweise 1,3-Bis(hydroxymethyl)-5,5-dimethylimidazolidin-2,4-dion (Nipaguard^{®} DMDMH).

Die Menge der Konservierungsmittel in den erfindungsgemäßen Zusammensetzungen beträgt im Allgemeinen von 0 bis 2,0 Gew.-%, bezogen auf das Gesamtgewicht der fertigen Zusammensetzungen.

Als Duftstoffe können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z. B. die Ionone, alpha-Isomethylionon und Methylcedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Als Duftstoffe können auch natürliche Riechstoffgemische verwendet werden, wie sie aus pflanzlichen oder tierischen Quellen zugänglich sind, z. B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Die Menge der Duftstoffe in den erfindungsgemäßen Zusammensetzungen beträgt im Allgemeinen von 0 bis 2 Gew.-%, bezogen auf das Gesamtgewicht der fertigen Zusammensetzungen.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide, wobei die letzteren gleichzeitig als Schaumstabilisatoren dienen, verwendet werden, die bevorzugt in Mengen von 0,01 bis 10,0 Gew.-%, besonders bevorzugt von 0,1 bis 5,0 Gew.-% und insbesondere bevorzugt von 0,5 bis 3,0 Gew.-% eingesetzt werden.

Die in den erfindungsgemäßen Zusammensetzungen enthaltenen Farbstoffe- und -pigmente, sowohl organische als auch anorganische Farbstoffe, können aus der entsprechenden Positivliste der Kosmetikverordnung bzw. der EG-Liste kosmetischer Färbemittel ausgewählt werden. Vorteilhaft eingesetzt werden auch Perlglanzpigmente, z. B. Fischsilber (Guanin/ Hypoxanthin-Mischkristalle aus Fischschuppen) und Perlmutt (vermahlene Muschelschalen), monokristalline Perlglanzpigmente wie z.B. Bismuthoxychlorid (BiOCl), Schicht-Substrat Pigmente, z. B. Glimmer/Metalloxid, silberweiße Perlglanzpigmente aus TiO₂, Interferenzpigmente (TiO₂, unterschiedliche Schichtdicke), Farbglanzpigmente (Fe₂O₃) und Kombinationspigmente (TiO₂/Fe₂O₃, TiO₂/Cr₂O₃, TiO₂/Berliner Blau, TiO₂/Carmin).

In einer bevorzugten Ausführungsform der Erfindung liegen die erfindungsgemäßen Zusammensetzungen in Form von Produkten zur Haar- und Hautreinigung wie Haarshampoos, Duschbäder, Handseifen und Gesichtsreiniger vor.

Die Erfindung wird durch die nachstehenden Beispiele näher erläutert.

### Beispiele

### Beispiele 1 und 2 sowie Vergleichsbeispiele 1 bis 4

Die im folgenden beschriebenen N-Acyl-N-methylglucamine wurden nach EP 0 550 637 aus den korrespondierenden Fettsäuremethylestern und N-Methylglucamin in Gegenwart von 1,2-Propylenglykol als Lösemittel hergestellt und als Feststoffe bestehend aus Aktivsubstanz und 1,2 Propylenglykol erhalten (alle Angaben in Gew.-%).

**Tabelle 1**

| Beispiel | Methylester | Aktivsubstanz (%) | 1,2 Propylenglykol (%) | Schmelzpunkt |
|---|---|---|---|---|
| Vergleichs-Beispiel 1 | C12/14 | 90 | 10 | 85 |
| 1 | C8/10 | 90 | 10 | 50 |

Die Viskositäten wurden mit einem Brookfield-Viskosimeter Model DV II, den Spindeln aus dem Spindelset RV bei 20 Umdrehungen / Minute und 20 °C gemessen. Es wurden die Spindeln 1 bis 7 aus dem Spindelset RV verwendet. Unter diesen Messbedingungen wurde Spindel 1 für Viskositäten von maximal 500 mPa·s, Spindel 2 für Viskositäten von maximal 1 000 mPa·s, Spindel 3 für Viskositäten von maximal 5 000 mPa·s, Spindel 4 für Viskositäten von maximal 10 000 mPa·s, Spindel 5 für Viskositäten von maximal 20 000 mPa·s, Spindel 6 für Viskositäten von maximal 50 000 mPa·s und Spindel 7 für Viskositäten von maximal 200 000 mPa·s gewählt.

Es wurden Tensidlösungen bestehend aus Natriumlaurylethersulfat (Genapol LRO liq., Clariant), Cocamidopropylbetain (Genagen CAB 818, Clariant) im Verhältnis 7 : 3 mit 15 Gew.-% Gesamtaktivsubstanz hergestellt und durch Zugabe von Kochsalz auf eine einheitliche Viskosität von etwa 5000 mPas angepasst.

In weiteren Versuchen wurden diese Tensidlösungen mit 1 Gew.-% zusätzlichem Zuckertensid beaufschlagt und ebenfalls auf ca. 5000 mPas Viskosität mit Salz angepasst.

Die Viskositäten wurden bei 20 °C ermittelt und die Beispielrezepturen dann auf 4 °C abgekühlt und die Viskosität erneut gemessen.

Das Ausmaß der Kältestabilität wurde als Reduktion der Viskosität beim Abkühlen in % ermittelt.

**Tabelle 2**

| Beispiel | Zuckertensid | Viskosität bei 20 °C | Viskosität bei 4 °C | Viskositäts-reduktion (%) | Aussehen der Lösung bei 4°C | Benötigte Salzmenge für 5000 mPas (%) |
|---|---|---|---|---|---|---|
| Beispiel 2 | Beispiel 1 | 4780 | 2400 | - 50 | klar | 1,2 |
| Vergleichs-beispiel 2 | Vergleichs-beispiel 1 | 4800 | 1330 | - 72 | trüb | 0,50 |
| Vergleichsbeispiel 3 | Plantacare 818 (Coco-Glucosid) | 5150 | 1580 | - 69 | trüb | 0,80 |
| Vergleichs-beispiel 4 | Keines | 4800 | 1410 | - 71 | trüb | 1,0 |

Wie aus der obenstehenden Tabelle hervorgeht, zeigt das erfindungsgemäße Beispiel 1 eine signifikant niedrigere Reduktion der Viskosität beim Abkühlen der Tensidlösung, wobei die Tensidlösung zusätzlich im Gegensatz zu den Vergleichsbeispielen klar bleibt.

## Patentansprüche

1. Verwendung von N-Methyl-N-acylglucaminen als Kältestabilisatoren in wässrigen Tensidlösungen, die ein oder mehrere anionische Tenside aus der Gruppe bestehend aus Alkylsulfaten und Alkylethersulfaten enthalten, sowie Betain-Tenside enthalten wobei mindestens 90 Gew.-% der N-Methyl-N-acylglucamine eine C₈-Acyl- oder C₁₀-Acylgruppe aufweisen.

2. Verwendung nach Anspruch 1, **dadurch gekennzeichnet, dass** die wässrigen Tensidlösungen ein Alkylsulfat und ein Alkylethersulfat als anionisches Tensid enthalten.

3. Verwendung nach Anspruch 1 oder 2, **dadurch gekennzeichnet, dass** die wässrigen Tensidlösungen ein lineares C₈-C₂₀-Alkylsulfat und/oder ein lineares C₈-C₂₀-Alkylethersulfat enthalten.

4. Verwendung nach Anspruch 3, **dadurch gekennzeichnet, dass** die wässrigen Tensidlösungen Laurylsulfat und/oder ein Laurylethersulfat enthalten.

5. Verwendung nach einem der Ansprüche 1 bis 4, **dadurch gekennzeichnet, dass** die wässrigen Tensidlösungen ein Acylamidopropylbetain oder ein Alkylbetain enthalten.

6. Zusammensetzungen enthaltend
(a) N-Methyl-N-acylglucamine, wobei mindestens 90 Gew.-% der N-Methyl-N-acylglucamine eine C₈-Acyl- oder C₁₀-Acylgruppe aufweisen, als Komponente (A),
(b) ein oder mehrere anionische Tenside aus der Gruppe bestehend aus Alkylsulfaten und Alkylethersulfaten als Komponente (B),
(c) ein oder mehrere Betain-Tenside als Komponente (C),
(d) gegebenenfalls weitere Tenside als Komponente (D),
(e) Wasser als Komponente (E),
(f) gegebenenfalls weitere Additive als Komponente (F).

7. Zusammensetzungen nach Anspruch 6 enthaltend
(a) 0,01 bis 5,0 Gew.-% der Komponente (A),
(b) 1,0 bis 20,0 Gew.-% der Komponente (B),
(c) 0,1 bis 10,0 Gew.-% der Komponente (C),
(d) 0 bis 5,0 Gew.-% der Komponente (D),
(e) 55,0 bis 98,89 Gew.-% der Komponente (E),
(f) 0 bis 5,0 Gew.-% der Komponente (F).

8. Zusammensetzungen nach Anspruch 6 oder 7 in Form von Haarshampoos, Duschbädern, Handseifen und Gesichtsreinigern.

9. Zusammensetzungen nach einem der Ansprüche 6 bis 8, **dadurch gekennzeichnet, dass** mindestens 95 Gew.-% der N-Methyl-N-acylglucamine eine C₈-Acyl- oder C₁₀-Acylgruppe aufweisen.

10. Zusammensetzungen nach einem der Ansprüche 6 bis 9, **dadurch gekennzeichnet, dass** die Zusammensetzungen weitere Tenside (D) enthalten und die weitere Tenside (D) kationische, nichtionische oder amphotere Tenside sind.

11. Zusammensetzungen nach einem der Ansprüche 6 bis 10, **dadurch gekennzeichnet, dass** die Zusammensetzungen amphotere Tenside enthalten und die amphotere Tenside N-(C₁₂-C₁₈)-Alkyl-β-aminopropionate oder N-(C₁₂-C₁₈)-Alkyl-β-iminodipropionate oder deren Alkali- und Mono-, Di- und Trialkylammonium-Salze sind.

12. Zusammensetzungen nach einem der Ansprüche 6 bis 11, **dadurch gekennzeichnet, dass** die Zusammensetzungen weitere Additive (F) enthalten und die weitere Additive (F) Konservierungsmittel, Duftstoffe, Farbstoffe oder Rückfettungsmittel sind.

13. Zusammensetzungen nach einem der Ansprüche 6 bis 12, **dadurch gekennzeichnet, dass** die Zusammensetzungen Perlglanzpigmente, monokristalline Perlglanzpigmente, Schicht-Substrat Pigmente, silberweiße Perlglanzpigmente aus TiO₂, Interferenzpigmente, Farbglanzpigmente oder Kombinationspigmente.

14. Zusammensetzungen nach einem der Ansprüche 12 bis 13, **dadurch gekennzeichnet, dass** die Zusammensetzungen ein Konservierungsmittel enthalten und das Konservierungsmittel aus der Gruppe bestehend aus Phenoxyethanol, Benzylalkohol, Parabene, Benzoesäure und Sorbinsäure ist.

15. Zusammensetzungen nach einem der Ansprüche 12 bis 13, **dadurch gekennzeichnet, dass** die Zusammensetzungen ein Konservierungsmittel enthalten und das Konservierungsmittel 1,3-Bis(hydroxymethyl)-5,5-dimethylimidazolidin-2,4-dion ist.

## Claims

1. The use of N-methyl-N-acylglucamines as cold stabilizers in aqueous surfactant solutions comprising one or more anionic surfactants from the group consisting of alkyl sulfates and alkyl ether sulfates, and comprising betaine surfactants, where at least 90% by weight of the N-methyl-N-acylglucamines contain a C₈-acyl or C₁₀-acyl group.

2. The use as claimed in claim 1, wherein the aqueous surfactant solutions comprise an alkyl sulfate and an alkyl ether sulfate as anionic surfactant.

3. The use as claimed in claim 1 or 2, wherein the aqueous surfactant solutions comprise a linear C₈-C₂₀-alkyl sulfate and/or a linear C₈-C₂₀-alkyl ether sulfate.

4. The use as claimed in claim 3, wherein the aqueous surfactant solutions comprise lauryl sulfate and/or a lauryl ether sulfate.

5. The use as claimed in any of claims 1 to 4, wherein the aqueous surfactant solutions comprise an acylamidopropyl betaine or an alkyl betaine.

6. A composition comprising
(a) N-methyl-N-acylglucamines where at least 90% by weight of the N-methyl-N-acylglucamines contain a C₈-acyl or C₁₀-acyl group as component (A),
(b) one or more anionic surfactants from the group consisting of alkyl sulfates and alkyl ether sulfates as component (B),
(c) one or more betaine surfactants as component (C),
(d) optionally further surfactants as component (D),
(e) water as component (E),
(f) optionally further additives as component (F).

7. The composition as claimed in claim 6, comprising
(a) 0.01% to 5.0% by weight of component (A),
(b) 1.0% to 20.0% by weight of component (B),
(c) 0.1% to 10.0% by weight of component (C),
(d) 0% to 5.0% by weight of component (D),
(e) 55.0% to 98.89% by weight of component (E),
(f) 0% to 5.0% by weight of component (F).

8. The composition as claimed in claim 6 or 7 in the form of a hair shampoo, shower gel, hand soap or face cleanser.

9. The composition as claimed in any of claims 6 to 8, wherein at least 95% by weight of the N-methyl-N-acylglucamines contain a C₈-acyl or C₁₀-acyl group.

10. The composition as claimed in any of claims 6 to 9, which comprises further surfactants (D) and wherein the further surfactants (D) are cationic, nonionic or amphoteric surfactants.

11. The composition as claimed in any of claims 6 to 10, which comprises amphoteric surfactants and wherein the amphoteric surfactants are N-(C₁₂-C₁₈)alkyl-β-aminopropionates or N- (C₁₂-C₁₈) alkyl-β-iminodipropionates or alkali metal and mono-, di- and trialkylammonium salts thereof.

12. The composition as claimed in any of claims 6 to 11, which composition comprises further additives (F) and wherein the further additives (F) are preservatives, fragrances, dyes or refatting agents.

13. The composition as claimed in any of claims 6 to 12, wherein the composition pearlescent pigments, monocrystalline pearlescent pigments, layer-substrate pigments, silver-white pearlescent pigments composed of TiO₂, interference pigments, color luster pigments or combination pigments.

14. The composition as claimed in either of claims 12 and 13, which comprises a preservative and wherein the preservative is from the group consisting of phenoxyethanol, benzyl alcohol, parabens, benzoic acid and sorbic acid.

15. The composition as claimed in either of claims 12 and 13, which comprises a preservative and wherein the preservative is 1,3-bis(hydroxymethyl)-5,5-dimethylimidazolidine-2,4-dione.

## Revendications

1. Utilisation de N-méthyl-N-acylglucamines en tant que stabilisants au froid dans des solutions aqueuses de tensioactifs qui contiennent un ou plusieurs tensioactifs anioniques choisis dans le groupe constitué par des alkylsulfates et des alkyléthersulfates, ainsi que des tensioactifs de type bétaïne, au moins 90 % en poids des N-méthyl-N-acylglucamines comportant un groupe acyle en C₈ ou acyle en C₁₀.

2. Utilisation selon la revendication 1, **caractérisée en ce que** les solutions aqueuses de tensioactifs contiennent en tant que tensioactif anionique un alkylsulfate et un alkyléthersulfate.

3. Utilisation selon la revendication 1 ou 2, **caractérisée en ce que** les solutions aqueuses de tensioactifs contiennent un alkyl(C₈-C₂₀) sulfate linéaire et/ou un alkyl(C₈-C₂₀)éthersulfate linéaire.

4. Utilisation selon la revendication 3, **caractérisée en ce que** les solutions aqueuses de tensioactifs contiennent du laurylsulfate et/ou un lauryléthersulfate.

5. Utilisation selon l'une quelconque des revendications 1 à 4, **caractérisée en ce que** les solutions aqueuses de tensioactifs contiennent une acylamidopropylbétaïne ou une alkylbétaïne.

6. Compositions contenant
(a) des N-méthyl-N-acylglucamines, au moins 90 % en poids des N-méthyl-N-acylglucamines comportant un groupe acyle en C₈ ou acyle en C₁₀, en tant que composant (A),
(b) un ou plusieurs tensioactifs anioniques choisis dans le groupe constitué par des alkylsulfates et des alkyléthersulfates, en tant que composant (B),
(c) un ou plusieurs tensioactifs de type bétaïne, en tant que composant (C),
(d) éventuellement d'autres tensioactifs en tant que composant (D),
(e) de l'eau en tant que composant (E),
(f) éventuellement d'autres additifs en tant que composant (F).

7. Compositions selon la revendication 6, contenant
(a) 0,01 à 5,0 % en poids du composant (A),
(b) 1,0 à 20,0 % en poids du composant (B),
(c) 0,1 à 10,0 % en poids du composant (C),
(d) 0 à 5,0 % en poids du composant (D),
(e) 55,0 à 98,89 % en poids du composant (E),
(f) 0 à 5,0 % en poids du composant (F).

8. Compositions selon la revendication 6 ou 7, sous forme de shampooings capillaires, gels de douche, savons pour les mains et produits de nettoyage pour le visage.

9. Compositions selon l'une quelconque des revendications 6 à 8, **caractérisées en ce qu'**au moins 95 % en poids des N-méthyl-N-acylglucamines comportant un groupe acyle en C₈ ou acyle en C₁₀.

10. Compositions selon l'une quelconque des revendications 6 à 9, **caractérisées en ce que** les compositions contiennent d'autres tensioactifs (D) et les autres tensioactifs (D) sont des tensioactifs cationiques, non ioniques ou amphotères.

11. Compositions selon l'une quelconque des revendications 6 à 10, **caractérisées en ce que** les compositions contiennent des tensioactifs amphotères, et les tensioactifs amphotères sont des N-alkyl(C₁₂-C₁₈)-β-aminopropionates ou des N-alkyl(C₁₂-C₁₈)-β-iminodipropionates ou leurs sels de métaux alcalins et de mono-, di- et trialkylammonium.

12. Compositions selon l'une quelconque des revendications 6 à 11, **caractérisées en ce que** les compositions contiennent d'autres additifs (F) et les autres additifs (F) sont des conservateurs, des parfums, des colorants ou des agents de regraissage.

13. Compositions selon l'une quelconque des revendications 6 à 12, **caractérisées en ce que** les compositions des pigments nacrés, des pigments nacrés monocristallins, des pigments à couche-substrat, des pigments nacrés blanc argenté à base de TiO₂, des pigments interférentiels, des pigments lustrants ou des pigments combinés.

14. Compositions selon l'une quelconque des revendications 12 et 13, **caractérisées en ce que** les compositions contiennent un conservateur et le conservateur est choisi dans le groupe constitué par le phénoxyéthanol, l'alcool benzylique, les parabènes, l'acide benzoïque et l'acide sorbique.

15. Compositions selon l'une quelconque des revendications 12 à 13, **caractérisées en ce que** les compositions contiennent un conservateur et le conservateur est la 1,3-bis(hydroxyméthyl)-5,5-diméthylimidazolidine-2,4-dione.
